# FASCICULE DE BREVET EUROPEEN

(11) **EP 2 380 538 B1**
(45) Date de publication et mention de la délivrance du brevet: **07.12.2016**
(21) Numéro de dépôt: 11003280.2
(22) Date de dépôt: 19.04.2011
(51) Int. Cl.: A61F 9/02

(54) **Masque de protection pour la pratique de sports de plein air**
Schutzbrille beim Ausüben von Outdoor-Sportarten
Protective goggles for the practice of outdoor sports

(30) Priorité: 22.04.2010 FR 1001730
(43) Date de publication de la demande: 26.10.2011
(73) Titulaire: SALOMON S.A.S., 74370 Metz-Tessy (FR)
(72) Inventeur: Renaud-Goud, Gilles, 74540 Gruffy (FR); Verhaeghe, Quentin, 74000 Annecy (FR)

(56) Documents cités:
- WO-A2-2004/034127
- US-A- 3 896 496
- US-A- 6 076 196
- US-A1- 2004 025 232

## Description

L'invention concerne les masques de protection, en particulier les masques de protection pour la pratique de sports de plein air, tels que le ski alpin, le vélo tout terrain ou le motocross.

Des masques de protection sont habituellement utilisés durant la pratique de certains sports de plein air pour protéger les yeux de l'utilisateur du vent, de l'eau, de la boue ou du rayonnement solaire. L'utilisation de tels masques est généralement favorisée par rapport à des lunettes de soleil à montures cerclées du fait du plus grand champ de vision continu procuré, en particulier dans la partie médiane. Par ailleurs, l'utilisation de tels masques est également favorisée pour permettre le port de lunettes correctrices tout en bénéficiant de la protection du masque.

Un tel masque de protection comprend habituellement une armature supportant un écran de protection transparent. L'écran de protection est généralement réalisé en matière synthétique. L'écran de protection est généralement composé d'une ou plusieurs parois continues et transparentes disposées en vis-à-vis des yeux de l'utilisateur. Une paroi extérieure de l'écran peut par exemple présenter des propriétés de filtrage des rayonnements solaires (par exemple pour réduire l'entrée des rayonnements ultraviolets dans le masque). Une paroi intérieure de l'écran peut par exemple présenter des propriétés pour limiter la condensation de la vapeur d'eau. L'écran est disposé dans une ouverture frontale de l'armature. L'armature forme un support périphérique de l'écran. L'armature supporte généralement une mousse périphérique venant au contact du visage de l'utilisateur, afin de s'adapter à sa morphologie. L'armature et la mousse maintiennent l'écran à distance du visage de l'utilisateur, de sorte qu'un volume intérieur est ménagé entre l'écran et le visage. Ce volume intérieur peut notamment recevoir des lunettes correctrices portées par l'utilisateur. Le masque de protection comprend généralement une sangle fixée de part et d'autre de l'armature. L'armature et la sangle forment ainsi une boucle, la sangle passant derrière le crâne de l'utilisateur pour assurer le maintien du masque sur le visage de l'utilisateur.

Un tel masque présente généralement des ouvertures ménagées dans l'armature ou dans l'écran pour mettre en communication le volume intérieur avec l'environnement extérieur. De telles ouvertures permettent d'évacuer la vapeur d'eau du volume intérieur et limitent ainsi la condensation sur l'écran en particulier lorsque l'utilisateur transpire. L'armature est généralement constituée d'une structure en matériaux polymères souples, afin d'amortir d'éventuels chocs contre le masque, de faciliter le montage de l'écran et d'améliorer l'adaptation du masque à la morphologie du visage de l'utilisateur. L'armature présente une rainure périphérique dans laquelle se loge la bordure de l'écran. Une telle rainure permet ainsi d'assurer le maintien en position de l'écran.

Un tel masque présente cependant des inconvénients. L'armature doit assurer à la fois le maintien de l'écran, la résistance aux chocs et l'adaptation à la forme du visage. Par conséquent, l'armature est réalisée dans un matériau flexible. L'armature présente une rainure relativement profonde pour loger la bordure de l'écran, et une largeur relativement profonde pour garantir une bonne retenue de l'écran. La hauteur et la largeur de l'armature limitent ainsi l'angle de vision sur l'ensemble du champ de vision. Un tel masque présente ainsi un champ de vision relativement réduit, et ce malgré une taille importante de l'écran.

Le document US 5,642,530 décrit un tel masque, l'armature y est réalisée en une seule pièce. Elle comprend une partie frontale dans laquelle est ménagée une profonde rainure et une paroi latérale dont la base est équipée d'une bande de mousse pour l'absorption de la transpiration.

De manière similaire, le document US 5,363,512, décrit un masque selon l'art antérieur dont l'armature, faite d'une seule pièce, est souple et flexible.

Le problème du champ de vision réduit est accentué lorsque le masque est équipé d'un écran double, ou écran à double paroi. Un exemple de ceci est montré dans le document EP 2 044 912. On peut voir à la figure 4 de ce document que seule une des deux parois de l'écran est encastrée dans une gorge profonde de l'armature, la deuxième paroi, la paroi intérieure est de dimension plus petite que la paroi extérieure et elle est fixée à cette dernière par l'intermédiaire d'un joint. Dans un tel cas, pour déterminer l'extension verticale du champ de vision, il faut mesurer la distance séparant la portion supérieure et la portion inférieure du joint. Une estimation des mesures sur le masque décrit dans le document EP 2 044 912 montre que l'extension verticale du champ de vision correspond à 46% de la hauteur totale de l'armature. Dans le cas hypothétique où le masque décrit dans ce document EP 2 044 912 serait équipé d'un écran simple paroi, l'extension verticale du champ de vision serait définie par la distance séparant les deux bords internes de l'armature et il correspondrait à 64% de l'extension verticale extérieure de l'armature.

Par conséquent, l'utilisation de matériaux flexibles pour la réalisation de l'armature a un effet très négatif sur le rapport champ de vision/encombrement du masque. Une étude des différents masques de ski présents sur le marché a montré que l'extension verticale du champ de vision est toujours inférieure à 73% de la hauteur totale du masque pour les masques actuels ayant un écran double paroi. Dans le cas où les armatures de ces masques seraient équipées d'écran simple paroi, le rapport serait toujours inférieur à 80%.

Par ailleurs, l'esthétique du masque est fortement influencée par la morphologie du visage de l'utilisateur. En effet, l'armature peut subir d'importantes déformations afin de s'adapter à la morphologie du visage de l'utilisateur, ce qui peut sensiblement modifier l'aspect du masque. De plus, la vision de l'utilisateur peut également être détériorée puisque ces déformations de l'armature sont répercutées sur l'écran. La forme de l'écran lors de son port peut ainsi être très différente de la forme conçue pour fournir une vision optimale en l'absence de contraintes sur le masque. Un choc contre le masque peut également générer une déformation excessive de l'écran aboutissant à sa rupture.

De plus, un tel masque ne permet pas une élimination suffisante de la buée sur l'écran, en particulier lorsque l'humidité ambiante est importante ou que l'utilisateur transpire abondamment. Cela s'avère particulièrement gênant lorsque l'utilisateur porte des verres correcteurs qui ne sont pas traités contre la buée. En accroissant la section des ouvertures d'aération, on réduit d'autant le champ de vision de l'utilisateur, on risque de générer un refroidissement désagréable sur le visage de l'utilisateur, sans pour autant obtenir des performances satisfaisantes d'évacuation de la vapeur d'eau. Certaines solutions techniques ont proposé de monter des ventilateurs. De tels ventilateurs nécessitent une alimentation électrique, alourdissent sensiblement le masque, accroissent sensiblement l'encombrement du masque et peuvent altérer le champ de vision procuré.

Un masque de protection oculaire tel que décrit dans le préambule de la revendication 1 est connu du document US-A-2004/0025232. L'invention vise à résoudre un ou plusieurs de ces inconvénients. L'invention porte ainsi sur un masque de protection oculaire pour la pratique de sports de plein air, comprenant :
- un écran de protection oculaire orienté sensiblement perpendiculairement à un axe de vision ;
- une armature périphérique sur laquelle l'écran est fixé, l'armature délimitant une ouverture obturée par l'écran, l'armature étant réalisée dans un matériau rigide ;
- une jupe présentant un deuxième anneau fixée sur l'armature et réalisée en matériau souple, la jupe présentant un premier anneau dont un tronçon médian est fixé de façon sensiblement immobile sur l'armature et présentant des tronçons latéraux s'étendant latéralement depuis le tronçon médian,
- une garniture fixée sur les tronçons latéraux et destinée à venir au contact avec le visage de l'utilisateur ;
- le volume intérieur délimité par l'écran, la jupe et la garniture, étant un espace fermé dès que le masque est posé contre le visage de l'utilisateur ;
- les tronçons latéraux étant mobiles axialement par rapport à l'armature de façon à modifier le volume intérieur et sont reliés au deuxième anneau par au moins un pontet.

Selon une variante, la jupe est réalisée dans un matériau présentant une dureté au moins trois fois inférieure à la dureté de l'armature.

Selon encore une variante, la jupe est réalisée dans un matériau présentant une dureté inférieure à 80 Shore A.

Selon une autre variante, le premier anneau présente un contour sensiblement identique au contour de l'armature. La jupe présente un deuxième anneau épousant la forme du contour de l'armature et fixé sur le contour de l'armature, le premier anneau étant relié au deuxième anneau par sa partie médiane, des dégagements étant ménagés entre les tronçons latéraux du premier anneau et le deuxième anneau.

Selon une variante, le masque comprend du textile respirant obturant les dégagements entre les tronçons latéraux du premier anneau et le deuxième anneau. Les tronçons latéraux du premier anneau sont reliés au deuxième anneau par des pontets traversant les dégagements.

Selon une autre variante, les pontets sont inclinés par rapport à l'axe du masque.

Selon une autre variante, le masque comprend une ceinture en mousse fixée sur le contour du premier anneau et destinée à venir au contact du visage d'un utilisateur.

Selon une variante, l'écran présente deux zones de vision destinées à venir en vis-à-vis des yeux de l'utilisateur et une partie médiane raccordant lesdites zones de vision, et l'armature présente une rainure logeant la bordure de l'écran, la rainure présentant une profondeur inférieure à 2,5 mm à la verticale desdites zones de vision de l'écran.

Selon une autre variante, le premier anneau présente deux tronçons médians à l'aplomb l'un de l'autre et à partir desquels s'étendent les tronçons latéraux, un tronçon médian étant destiné à venir en vis-à-vis du front de l'utilisateur, l'autre tronçon médian étant destiné à venir en vis-à-vis du nez de l'utilisateur.

D'autres caractéristiques et avantages de l'invention ressortiront clairement de la description qui en est faite ci-après, à titre indicatif et nullement limitatif, en référence aux dessins annexés, dans lesquels :
- la figure 1 est une vue en perspective d'un masque selon un mode de réalisation de l'invention ;
- la figure 2 est une vue de dessus du masque de la figure 1 ;
- la figure 3 est une vue de face d'une partie du masque de la figure 1 ;
- la figure 4 est une vue en coupe de côté d'un mode de fixation d'un écran sur une armature ;
- la figure 5 est une vue en coupe de côté de l'armature au niveau d'une zone de fixation avec l'écran ;
- la figure 6 est une vue en coupe de côté d'un perfectionnement du masque ;
- la figure 7 illustre un écoulement d'air vers l'intérieur du masque de la figure 6;
- la figure 8 est une vue de face de l'armature d'une variante de masque ;
- la figure 9 est une vue en perspective de l'armature de la figure 8 sur laquelle est fixé un déflecteur ;
- la figure 10 est une vue en perspective du déflecteur de la figure 9 ;
- la figure 11 est une vue en coupe de dessus illustrant des écoulements d'air générés par le déflecteur ;
- la figure 12 est une vue en perspective d'une armature selon une variante de masque ;
- la figure 13 est une vue en perspective de la bordure d'un écran destiné à être fixé à l'armature de la figure 12 ;
- la figure 14 est une vue en coupe de côté de la fixation de l'écran de la figure 13 sur l'armature de la figure 12.
- la figure 15 est une vue en coupe partielle suivant le plan C, visible à la figure 1,
- la figure 16 est une vue en coupe verticale du masque de la figure 1 au niveau de la zone centrale d'une des deux zones de vision,
- la figure 17 est une vue de côté de la jupe du masque de la figure 1.

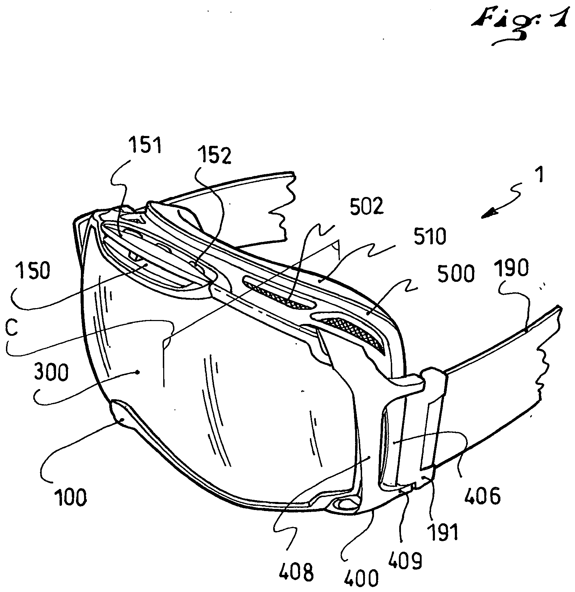

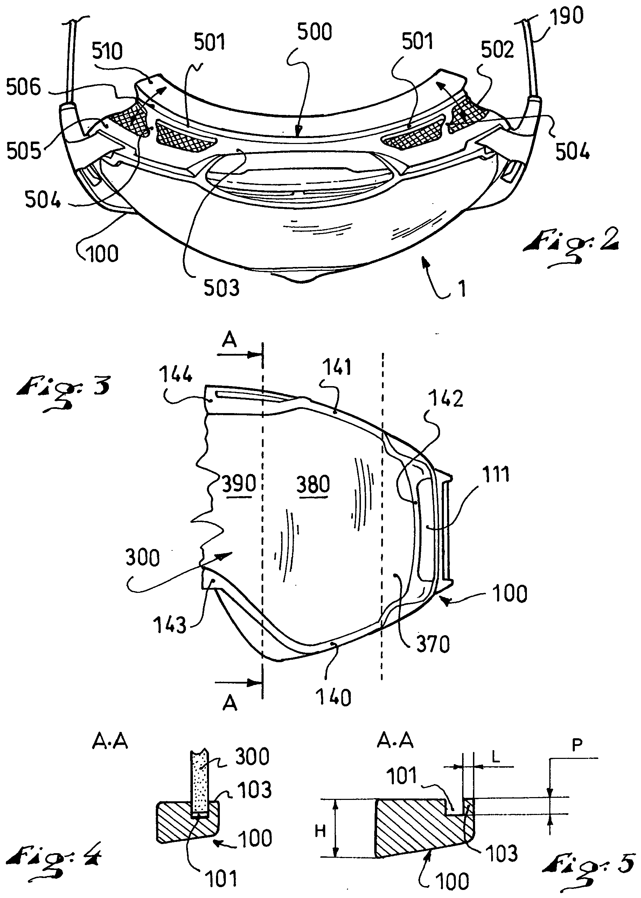

La figure 1 est une vue en perspective d'un masque 1 selon un mode de réalisation de l'invention. La figure 2 est une vue de dessus schématique de ce masque 1. Le masque 1 comprend une armature périphérique 100. L'armature 100 délimite une ouverture 102. L'ouverture 102 est obturée par un écran de protection oculaire 300. L'écran 300 est fixé à l'armature 100. Une sangle 190 est fixée aux extrémités latérales du masque 1. La sangle 190 et l'armature 100 forment une boucle destinée à entourer le crâne de l'utilisateur pour assurer son maintien en place.

La figure 3 est une vue de face de la moitié gauche de l'armature 100 munie de l'écran 300. L'écran de protection 300 obture l'ouverture 102 afin de protéger les yeux de l'utilisateur d'agressions extérieures telles que le vent, la neige ou l'eau. L'écran 300 est transparent et peut présenter de façon connue en soi des propriétés de filtration lumineuse, afin d'assurer la protection des yeux de l'utilisateur contre le rayonnement solaire. L'écran 300 pourra par exemple assurer la filtration des ultraviolets.

L'écran 300 présente deux zones de vision 380 destinées à venir en vis-à-vis des yeux de l'utilisateur. Les deux zones de vision 380 sont raccordées ensemble par une partie médiane 390. La partie médiane 390 est destinée à venir à l'aplomb du nez de l'utilisateur. De façon connue en soi, l'écran 300 peut former une paroi continue s'étendant entre les zones de vision 380 en passant par la partie médiane 390, afin d'optimiser l'angle de vision de l'utilisateur au niveau de la partie médiane. L'écran 300 présente également des zones de vision périphérique 370 ménagées dans ses extrémités latérales.

L'armature 100 présente une partie médiane supérieure 144, des parties supérieures latérales 141, des parties d'extrémités latérales 142, des parties inférieures latérales 140 et une partie inférieure médiane 143. La partie médiane inférieure 143 est échancrée pour le passage du nez de l'utilisateur.

Les figures 4 et 5 sont des vues en coupe de côté de l'armature 100 au niveau d'une zone de vision, respectivement en présence et en l'absence de l'écran 300. L'armature 100 présente une rainure 101 logeant la bordure de l'écran 300. L'écran 300 peut avantageusement être fixé à l'armature 100 par emboîtement dans la rainure 101. La rainure 101 peut avantageusement s'étendre tout autour de l'écran 300, sur toute la périphérie de l'ouverture 102, pour assurer le maintien optimal de l'écran 300.

A la verticale desdites zones de vision 380 de l'écran 300, c'est-à-dire dans la partie inférieure 140 et la partie supérieure 141, la rainure 101 présente une profondeur P inférieure à 2,5 mm, avantageusement inférieure à 2 mm et potentiellement inférieure à 1,5 mm. Ainsi, l'angle de vision de l'utilisateur à la verticale des zones 380 pourra être sensiblement amélioré, puisque la hauteur de l'écran 300 pourra être accrue pour un encombrement donné du masque 1. Avantageusement, la rainure 101 présente une telle profondeur P sur toute la périphérie de l'ouverture 102, afin d'optimiser l'angle de vision de l'utilisateur sur l'ensemble de son champ de vision.

L'utilisation d'une telle profondeur de la rainure 101 est notamment rendue possible du fait de l'utilisation d'une armature 100 réalisée dans un matériau rigide. Une rainure 101 de moindre profondeur peut ainsi être utilisée pour fixer l'écran 300 du fait de la moindre déformation subie par l'armature 100. Par ailleurs, une armature 100 rigide permet d'utiliser un écran 300 en matériau relativement rigide, l'écran n'ayant pas à subir d'importantes déformations. Avantageusement, le matériau choisi pour réaliser l'armature 100 présente un module d'élasticité supérieur à 1500 MPa, de préférence supérieur à 2500 MPa, voire même supérieur à 4000 MPa. Un tel matériau pourra être réalisé en matière synthétique, renforcée ou non. L'armature 100 pourra par exemple être réalisée en polyamide, en polyuréthane ou tout autre matériau plastique. Un polyamide tel que celui distribué sous la référence commerciale Grilamid TR55 ou Grilamid TR90 peut par exemple être utilisé. Par ailleurs, l'armature 100 pourra présenter une dureté supérieure à 70 Shore D, de préférence supérieure à 80 Shore D.

Du fait de sa rigidité, l'armature 100 peut présenter d'autres mesures constructives permettant d'accroître l'angle de vision de l'utilisateur.

Comme illustré aux figures 4 et 5, l'armature 100 présente un rebord 103 définissant une butée frontale de la rainure 101. Le rebord 103 présente une épaisseur L avantageusement inférieure à 2,5 mm, de préférence inférieure à 2 millimètres, voire inférieure ou égale à 1,5 mm, à la verticale de la zone de vision 380, dans les parties 140 et 141. Bien entendu, le rebord 103 peut présenter une telle valeur d'épaisseur sur toute la périphérie de l'ouverture 102.

Pour maximiser encore l'angle de vision de l'utilisateur, la section de l'armature 100 dans les parties 140 et 141 présente une hauteur H inférieure à 6 mm, de préférence inférieure à 5 mm, voire inférieure ou égale à 4 mm.

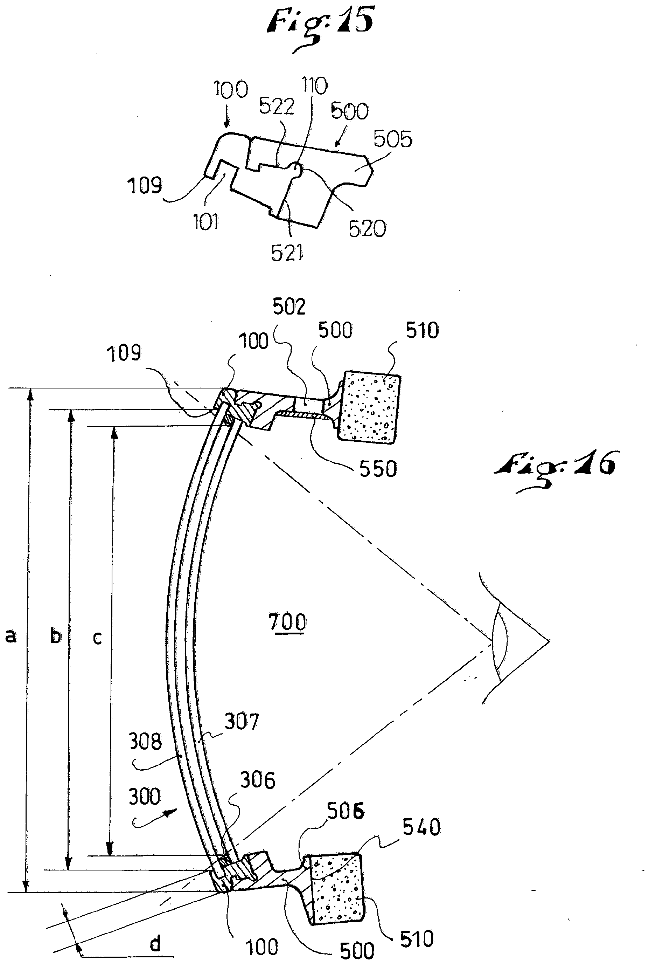

La figure 16 montre le masque de la figure 1 en coupe selon le plan C. Le plan C correspond à un plan vertical coupant le masque sensiblement au centre d'une des zones de vision. La mesure de la portion libre de l'écran au niveau de ce plan permet de déterminer l'extension verticale du champ de vision. Cette mesure est représentée par la flèche "c" sur la figure. Deux autres mesures sont faites au niveau du plan C, la hauteur de l'armature "a" et la distance séparant les deux bords internes 109 des rebords 103 de l'armature "b". Plus précisément, la mesure "a" correspond à la hauteur totale de l'armature. En effet, bien que cela ne soit pas le cas pour le mode de réalisation décrit à la figure 1, on peut imaginer que la hauteur totale du masque soit légèrement supérieure à la hauteur de l'armature.

Dans un masque selon l'invention, l'extension verticale au niveau du centre des zones de vision est supérieure à 78% de la hauteur du masque mesurée au même niveau. De préférence, on choisira de porter ce rapport à 80% par exemple en diminuant la profondeur de la gorge et/ou en augmentant la rigidité de l'armature.

Dans le masque représenté aux figures 1 et 16, l'extension verticale du champ de vision "c" est sensiblement supérieure à 86%.

D'autre part, d'un point de vue esthétique, la faible profondeur de la gorge recevant l'écran a une conséquence très notable : un masque dont l'écran représente la plus grande partie de la face frontale. En effet, la distance "b" séparant les bords internes de l'armature est relativement grande par rapport à la hauteur "a" du masque. Dans un masque selon l'invention, "b" est supérieur à 85% de la valeur "a", de préférence supérieur à 88%. Pour exemple, le masque représenté aux figures 1 et 16 présente un rapport entre b et a légèrement supérieur à 93%. Il est à noter que le rapport b/a correspondrait au rapport entre extension verticale du champ de vision sur la hauteur du masque dans le cas où l'écran n'aurait qu'une seule paroi.

Le caractère "large champ de vision" du masque selon l'invention est également donné par le fait que la hauteur "d" de l'armature au niveau du plan C est inférieure à 5 mm, de préférence à 3 mm comme dans le mode de réalisation décrit ici.

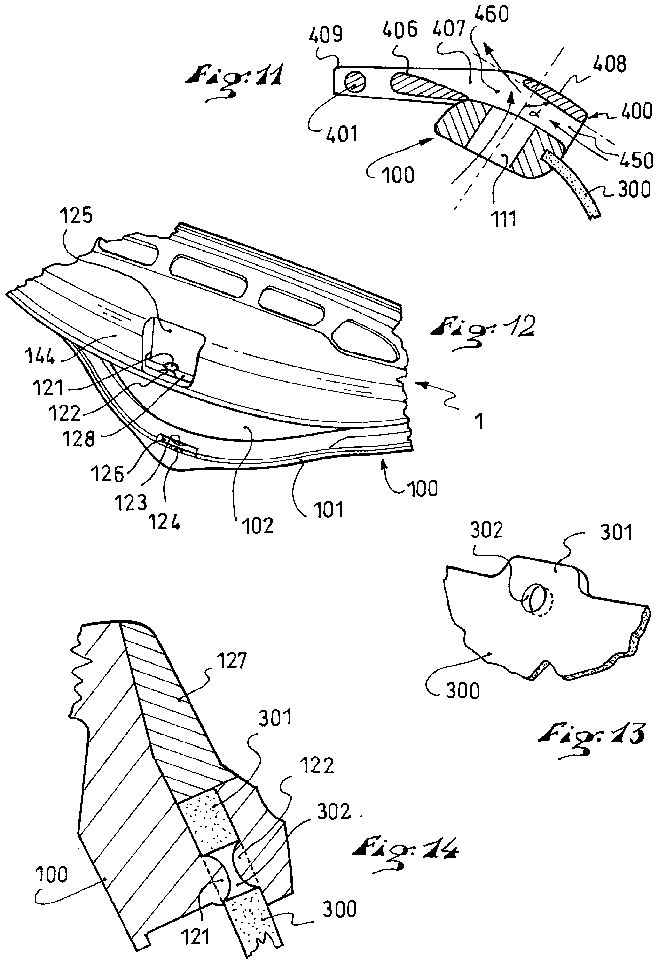

Les figures 12 à 13 illustrent une variante permettant d'améliorer un mode de fixation par emboîtement de l'écran 300 dans l'armature 100. Dans cet exemple, l'écran 300 présente des orifices respectivement dans sa bordure médiane supérieure et dans sa bordure médiane inférieure. La figure 13 illustre une partie de l'écran 300 au niveau de son orifice supérieur 302. La bordure de l'écran 300 forme une excroissance 301 à l'aplomb de l'orifice 302.

L'armature 100 présente un orifice 128 dans sa partie supérieure 144. L'orifice 128 est disposé dans l'alignement de la rainure 101. L'orifice 128 est avantageusement ménagé au niveau d'un renfoncement 125 de l'armature 100. Le renfoncement 125 peut-être conformé pour recevoir une pastille 127 comportant un logo. Des renflements 121 et 122 sont ménagés de part et d'autre de l'ouverture 128 en saillie à l'intérieur de l'ouverture. L'armature 100 présente également un orifice 126 dans sa partie inférieure. Des renflements 123 et 124 sont ménagés de part et d'autre de l'ouverture 126 en saillie à l'intérieur de l'ouverture.

Comme illustré à la figure 14, lors de l'introduction de la bordure de l'écran 300 dans la rainure 101, l'excroissance 301 se loge dans l'ouverture 128. Les renflements 121 et 122 viennent alors s'encliqueter dans l'orifice 302 de l'écran 300. La fixation mécanique de l'écran 300 sur l'armature 100 est ainsi améliorée. La pastille 127 est disposée dans le renfoncement 125 et permet de masquer l'ouverture 128 et l'excroissance 301.

Selon un autre aspect de l'invention représenté à la figure 1, le masque 1 présente une jupe 500 déformable fixée sur l'armature 100. La jupe 500 s'étend à l'arrière de l'armature 100 et délimite une deuxième ouverture placée dans l'alignement de l'ouverture 102. La jupe 500 est réalisée en matériau souple. La jupe 500 est formée dans un matériau distinct de celui de l'armature et présentant une dureté très inférieure à celle de l'armature 100. La jupe 500 permet de définir le volume intérieur du masque derrière l'écran, entre ce dernier et le visage de l'utilisateur. La jupe 500 est destinée à pouvoir se déformer suffisamment pour s'adapter à la morphologie du visage de l'utilisateur ou à absorber des chocs appliqués sur l'armature 100. Une telle jupe 500 s'avère donc particulièrement appropriée en combinaison avec l'armature rigide 100 décrite précédemment, qui présente une faculté réduite d'adaptation à la morphologie du visage et d'absorption des chocs. La jupe pourra être réalisée dans un matériau similaire de celui qui est utilisé pour la réalisation de l'armature des masques de ski de l'art antérieur.

La jupe 500 présente un anneau 505 fixé sur tout le contour de l'armature 100. La jupe 500 présente par ailleurs un autre anneau 506, disposé à l'arrière de l'anneau 505. Avantageusement, les anneaux 505 et 506 sont réalisés d'un seul tenant. L'anneau 506 présente un contour sensiblement identique à celui de l'anneau 505. L'anneau 506 présente donc un contour sensiblement identique à celui de l'armature 100, ce qui permet d'éviter que l'armature 100 ne vienne en contact avec le visage de l'utilisateur lors d'un choc. L'anneau 505 présente un tronçon médian 503 par lequel il est fixé à l'armature 100. En pratique, le tronçon médian 503 est commun à l'anneau 505 et à l'anneau 506. L'anneau 506 présente en pratique deux tronçons médians, l'un au niveau du front, l'autre au niveau du nez de l'utilisateur. Un tronçon médian 503 est ainsi interposé entre le front de l'utilisateur et l'armature 100, l'autre tronçon médian 503 étant interposé entre le nez de l'utilisateur et l'armature 100, les tronçons médians 503 permettant d'assurer le maintien en position du masque rapport au visage de l'utilisateur. L'anneau 506 présente des tronçons latéraux 501, s'étendant latéralement depuis les tronçons médians 503.

Comme illustré par les flèches de la figure 2, des tronçons latéraux 501 sont mobiles axialement par rapport à l'armature 100 de façon à modifier le volume intérieur 700. La mobilité des tronçons latéraux 501 combinée à la souplesse du matériau de la jupe 500 permet au masque 1 de s'adapter à la morphologie du visage de l'utilisateur. À cet effet, l'anneau 506 pourra présenter un rayon de courbure autour d'un axe vertical inférieur au rayon de courbure usuel du visage des utilisateurs, pour garantir que l'anneau 506 épouse la forme du visage de l'ensemble des utilisateurs potentiels. La déformation de la jupe permet d'augmenter le rayon de courbure de l'anneau 506. En pratique, un dégagement est ménagé latéralement entre les tronçons latéraux 501 et l'anneau 505, pour permettre une certaine amplitude de déformation et d'absorption de choc. A contrario, le tronçon médian 503 est sensiblement fixe par rapport à l'armature 100. Ainsi, lorsqu'il est compressé axialement, la partie arrière du tronçon médian subit un déplacement limité, très inférieur au déplacement subi par les tronçons latéraux pour une compression axiale de même amplitude.

Dans l'exemple, le tronçon médian 503 forme un bloc de matière continu entre l'armature 100 et les tronçons latéraux 501. Ainsi, les tronçons médians 503 permettent d'assurer un bon maintien de l'armature 100, sans altérer le confort de port par l'utilisateur. L'utilisation d'un matériau souple pour la jupe 500 permet de ne pas répercuter brutalement un choc sur le nez ou le front de l'utilisateur par l'intermédiaire des tronçons médians 503.

Bien que l'exemple illustré à la figure 2 comprend deux anneaux superposés axialement, on peut éventuellement utiliser un unique anneau 506 fixé sur l'armature 100 par son tronçon médian 503 et dont les tronçons latéraux 501 sont espacés de l'armature 100.

Pour limiter le débattement des tronçons latéraux 501 vers l'arrière, des pontets supérieurs 504 relient avantageusement les tronçons latéraux 501 à l'anneau 505. De tels pontets supérieurs 504 améliorent également le maintien en position de l'armature 100 par rapport au visage de l'utilisateur, ainsi que la résistance du masque 1 à l'usure. Pour faciliter les mouvements des tronçons 501, les pontets supérieurs 504 sont avantageusement inclinés par rapport à l'axe du masque, ce qui favorise leur flexion.

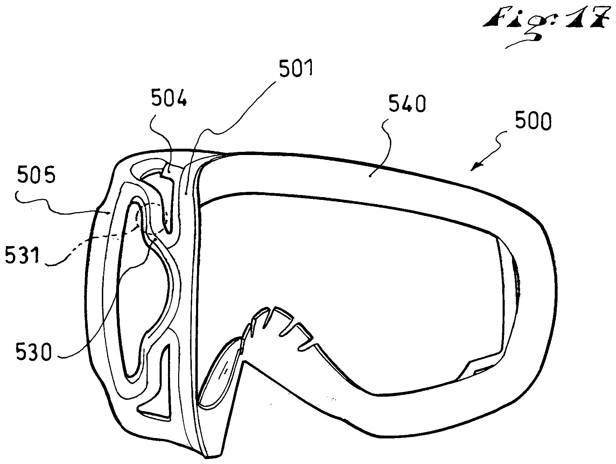

La figure 17 montre une vue latérale de la jupe 500 permettant de voir les portions de celle-ci qui sont cachées par le volet extérieur 408. On peut voir que le tronçon latéral 501 est relié à l'anneau 505 par l'intermédiaire de deux pontets latéraux 530. Ces pontets latéraux ont une forme qui rend possible le rapprochement entre l'anneau 505 et le tronçon latéral 501. En effet, la portion centrale 531 du pontet latéral 530 peut aisément se déformer car elle travaille en flexion lorsque le tronçon latéral 501 se rapproche de l'anneau 505.

Pour améliorer encore l'adaptation du masque 1 à la morphologie du visage de l'utilisateur, le masque 1 présente avantageusement une garniture 510. La garniture 510 forme une ceinture fixée à l'arrière de l'anneau 506 sur son contour 540. La garniture 510 définit une troisième ouverture placée dans l'alignement axial de l'ouverture 102 et elle est destinée à venir en contact avec le visage de l'utilisateur. La garniture 510 peut être réalisée en mousse, de façon connue en soi.

L'armature 100, l'écran 300, la jupe 500 et la garniture 510 délimitent ainsi un volume intérieur du masque 1. L'utilisateur pourra par exemple disposer des verres correcteurs dans ce volume. Le volume intérieur du masque 1 sert également à limiter l'apparition d'un phénomène de condensation sur la face intérieure de l'écran 300.

Le dégagement 502 entre les tronçons latéraux 501 et l'anneau 505 est avantageusement obturé par un textile respirant 550, afin de permettre l'évacuation de l'humidité hors du masque tout en empêchant la pénétration de corps étrangers. Par tissu respirant, on entend un tissu pouvant être traversé par de l'air ou de la vapeur d'eau.

La jonction entre les tronçons latéraux 501 et l'anneau 505 et/ou l'armature 100 peut également être assurée par un soufflet logé dans les dégagements, ou par la présence de chevrons dans les dégagements.

On peut également envisager que les dégagements entre les tronçons latéraux 501 et l'anneau 505 et/ou l'armature 100 soient obturés par une mousse, permettant l'évacuation de l'humidité depuis l'intérieur du masque.

Avantageusement, le matériau de la jupe 500 présente une dureté au moins trois fois inférieure à la dureté de l'armature 100, voire au moins cinq fois inférieure. La dureté du matériau de la jupe 500 pourra par exemple être inférieure ou égale à 80 Shore A, de préférence inférieure ou égale à 70 Shore A. La dureté de la jupe 500 pourra par exemple être comprise entre 55 et 70 Shore A. La jupe 500 pourra être réalisée en tout matériau approprié, par exemple en polyuréthane.

La figure 15 montre une vue en coupe partielle de l'assemblage entre l'armature 100 et la jupe 500. La jupe est pourvue sur une grande partie de la surface extérieure de l'anneau 505 d'une gorge 520, tandis que l'armature 100 se prolonge sur sa face opposée à celle qui est équipée de la rainure 101 d'un bourrelet 110 de forme complémentaire à celle de la gorge 520. Les dimensions intérieures de la gorge 520, et notamment, l'angle que font entre elles les deux faces planes 521,522 qui prolongent la portion de section circulaire de la gorge 520, sont légèrement inférieures à celles du bourrelet 110. Ainsi, compte tenu de la différence de souplesse entre les matériaux de la jupe 500 et de l'armature 100 une liaison par clipage est réalisée entre ces deux éléments. Cette liaison par clipage peut être complétée par collage et/ou de l'interface entre la jupe 500 et l'armature 100 au niveau des faces planes 521, 522.

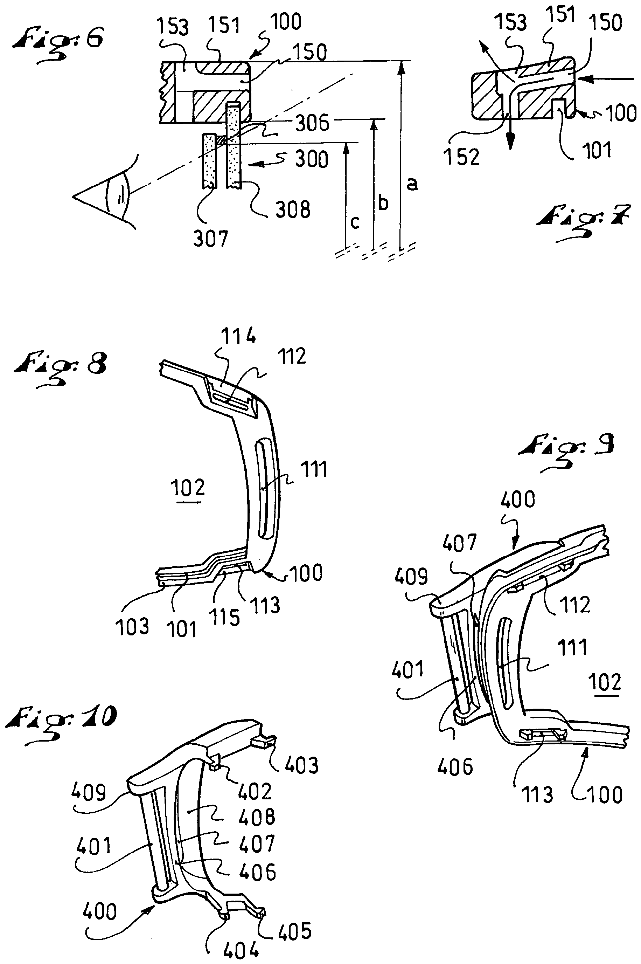

Comme illustré à la figure 1, la partie de l'écran 300 obturant l'ouverture 102 est dépourvue d'orifice traversant. Ainsi, l'angle de vision de l'utilisateur n'est pas affecté par de tels orifices. L'absence de tels orifices s'avère particulièrement avantageuse lorsque l'écran 300 présente plusieurs parois superposées, comme illustré à la figure 6. Dans l'exemple de la figure 6, deux parois 307 et 308 sont superposées axialement. La paroi 308 est fixée dans la rainure 101 et est par exemple destinée à absorber les chocs et à filtrer une partie du rayonnement solaire. La paroi 307 est fixée sur la paroi 308 par l'intermédiaire d'un joint périphérique 306. Le joint périphérique 306 permet de garantir une étanchéité entre les parois 307 et 308. Des orifices d'aération traversant l'écran devraient en pratique traverser la paroi 308 et devraient être situés à l'extérieur du joint 306 afin de garantir l'étanchéité entre les parois 307 et 308. En l'absence de tels orifices, le joint 306 peut être accolé au plus près de la rainure 101 de l'armature 100, ce qui accroît l'angle vision de l'utilisateur.

L'armature 100 présente avantageusement une ouverture frontale 150 ménagée dans sa partie 144, disposée au-dessus de la partie médiane 390 de l'écran 300. Une telle ouverture frontale 150 disposée dans la partie médiane de l'armature 100 permet de ne pas affecter la vision de l'utilisateur dans les zones de vision 380. L'armature 100 présente un déflecteur frontal 151 disposé sur sa face frontale. Le déflecteur frontal 151 délimite la partie supérieure de l'ouverture 150. Des conduits 152 sont disposés à l'arrière de l'ouverture 150. Les conduits 152 mettent en communication le volume intérieur du masque avec l'extérieur. Les entrées externes des conduits 152 communiquent avec l'ouverture 150. Les conduits 152 débouchent dans le volume intérieur du masque à l'arrière de l'écran 300. Une ouverture 153 est ménagée à l'arrière du déflecteur frontal 151, à l'aplomb des conduits 152.

Comme illustré à la figure 7, de l'air s'engouffre dans l'ouverture 150 lorsque l'utilisateur se déplace. Le déflecteur frontal 151 canalise l'écoulement formé dans l'ouverture 150 de sorte qu'un écoulement est formé vers l'intérieur du masque 1 à travers des conduits 152. L'ouverture 153 permet de limiter la surpression à l'intérieur du masque en laissant un écoulement ressortir vers le haut du masque. La partie frontale du déflecteur frontal 151 est avantageusement relevée par rapport à sa partie arrière, de façon à guider de l'air entrant par l'ouverture 150 vers les conduits 152. Cette structure d'introduction d'air à l'intérieur du masque 1 est avantageusement combinée aux ouvertures 111 et au déflecteur latéral 400 (détaillés par la suite) pour générer un écoulement allant des conduits 152 aux ouvertures 111, ce qui favorise l'évacuation de la vapeur d'eau hors du masque 1.

Selon un autre aspect de l'invention illustré de façon plus détaillée aux figures 8 à 11, l'armature 100 présente un orifice 111 mettant en communication l'intérieur du masque avec l'extérieur du masque. L'orifice 111 débouche à l'intérieur du masque 1 à l'arrière de l'écran 300. En l'occurrence, l'orifice 111 est ménagé dans la partie d'extrémité latérale 142 de l'armature 100. L'orifice 111 étant disposé au niveau d'une extrémité latérale de l'armature 100, il ne réduit pas l'angle de vision de l'utilisateur dans la partie périphérique. Afin d'optimiser l'aspiration sans altérer le champ de vision périphérique, l'orifice 111 s'étend sur la majeure partie de la hauteur de l'armature 100. Le masque 1 comprend un déflecteur latéral 400 fixé sur l'armature 100. Le déflecteur latéral 400 est disposé en vis-à-vis de l'orifice 111. Le déflecteur latéral 400 est configuré pour canaliser un écoulement d'air provenant de l'avant du masque lorsque l'utilisateur se déplace, de façon à former un écoulement d'air tangentiel à l'ouverture externe de l'orifice 111, de sorte que l'écoulement d'air formé induit une dépression par effet venturi au niveau de l'ouverture externe de l'orifice 111. La dépression permet de former un écoulement d'air allant de l'intérieur du masque vers l'extérieur à travers l'orifice 111.

Le déflecteur latéral 400, appelé encore diffuseur, présente un volet extérieur 408 disposé en face de l'orifice 111 légèrement en avant de celui-ci. Le volet extérieur 408 s'étend sur toute la hauteur de l'orifice 111. Le volet extérieur 408 présente une surface interne guidant l'écoulement d'air formé tangentiellement à l'ouverture externe de l'orifice 111. Le déflecteur latéral 400 présente un volet intérieur 406 placé à l'arrière du volet extérieur 408. Le volet intérieur 406 s'étend vers l'arrière depuis le bord arrière de l'ouverture extérieure du premier conduit. En l'occurrence, l'armature 100 est affleurante avec le volet intérieur 406 pour former une surface de guidage sensiblement continue de la partie aval de l'écoulement d'air formé. Les volets 406 et 408 sont séparés par un passage 407 traversé par l'écoulement d'air formé. De manière générale, le déflecteur latéral 400 définit un passage 407 entre le volet extérieur 408 d'une part et l'armature 100 et le volet intérieur 406 d'autre part. Le passage 407 assure un écoulement d'air entre un orifice d'admission 450 et un orifice d'échappement 460. L'orifice d'admission 450 est ouvert en direction de la face avant du masque de façon que en cours d'utilisation (i.e. lorsque le sportif se déplace vers l'avant), il y ait pénétration forcée d'air dans le passage 407. L'orifice d'échappement 460 est, quant à lui, ouvert en direction de l'arrière du masque de façon que l'écoulement puisse être très fluide et avec un débit important entre l'orifice d'admission 450 et l'orifice d'échappement 460. L'orifice 111, met en communication l'intérieur du masque avec le passage 407. En effet, l'orifice 111 débouche dans le passage 407. Le passage 407 présente une section réduite au niveau de l'orifice 111, et une section élargie au niveau de son extrémité aval au niveau de l'orifice d'échappement. La combinaison des volets 406 et 408 forme une constriction de l'écoulement d'air formé, sensiblement au niveau de l'ouverture extérieure de l'orifice 111.

L'orifice 111 du mode de réalisation illustré est délimité par un conduit formé dans l'armature 100. Ce conduit s'étend entre l'intérieur et l'extérieur du masque 1 selon une direction sensiblement normale à la surface interne de guidage du volet extérieur 408. L'angle entre la direction d'extension du conduit et la surface interne de guidage du volet extérieur 408 est représenté par l'angle α à la figure 11.

Le déflecteur latéral 400 de l'exemple est fixé par encliquetage sur l'armature 100. À cet effet, le déflecteur latéral 400 présente une première patte au niveau de son extrémité supérieure munie de crochets 402 et 403. Le déflecteur latéral 400 présente également une deuxième patte au niveau de son extrémité inférieure munie de crochets 404 et 405. Les crochets 402, 403 et 404, 405 traversent des ouvertures respectives 112 et 113 de l'armature 100. Les ouvertures 112 et 113 sont ménagées respectivement dans la partie supérieure et dans la partie inférieure de la partie 142 de l'armature 100. Les crochets 402 à 405 viennent en prise avec une face arrière de l'armature 100. L'armature 100 présente avantageusement des renfoncements 114 et 115 pour recevoir les pattes de fixation du déflecteur latéral 400. L'armature 100 présente avantageusement une section plus importante au niveau des ouvertures 112 et 113.

Le déflecteur latéral 400 comporte avantageusement des moyens de fixation pour la sangle 190. Le déflecteur latéral 400 présente un étrier 409 placé à l'arrière du volet intérieur 406. La fixation de la sangle 190 est avantageusement dans l'alignement du volet intérieur 406. Un arbre 401 s'étend verticalement dans l'étrier et forme un support pour la fixation de la sangle 190. Dans l'exemple illustré à la figure 1, un connecteur 191 est monté pivotant sur l'arbre 401. La sangle 190 est sertie dans le connecteur 191.

Dans l'exemple illustré, l'écran 300 présente un rayon de courbure inférieur à 400 mm, de préférence inférieure à 300 mm. Le conduit délimitant l'orifice 111 s'étend selon une direction normale à la zone 370 de l'écran, c'est-à-dire la zone accolée à l'orifice 111.

Comme illustré à la figure 11, l'effet venturi généré par l'écoulement d'air formé dans le passage 407 génère une dépression dans l'orifice 111, de sorte que de l'air est aspiré de l'intérieur du masque vers l'extérieur du masque. L'évacuation de la buée présente à l'intérieur du masque 1 est ainsi améliorée. Afin de favoriser l'extraction d'air hors du masque, le conduit délimitant l'orifice 111 s'étend avantageusement selon une direction formant un retour par rapport à l'écoulement formé. Cette direction formant un retour peut être identifiée par un angle α aigu entre la surface de guidage du volet extérieur 408 et la direction d'extension du conduit délimitant l'orifice.

## Revendications

1. Masque de protection oculaire (1) pour la pratique de sports de plein air, comprenant:
- un écran de protection oculaire (300) orienté sensiblement perpendiculairement à un axe de vision ;
- une armature périphérique (100) sur laquelle l'écran est fixé, l'armature délimitant une ouverture (102) obturée par l'écran (300), l'armature étant réalisée dans un matériau rigide ;
- une jupe (500) présentant un deuxième anneau (505) fixé sur l'armature (100) et un premier anneau (506) dont un tronçon médian (503) est fixé de façon sensiblement immobile sur l'armature (100) et présentant des tronçons latéraux (501) s'étendant latéralement depuis le tronçon médian, la jupe étant réalisée en matériau souple ;
- une garniture (510) fixée sur les tronçons latéraux (501) et destinée à venir au contact avec le visage de l'utilisateur ;
- le volume intérieur (700) délimité par l'écran (300), la jupe (500) et la garniture (510), étant un espace fermé dès que le masque est posé contre le visage de l'utilisateur ;
**caractérisé en ce que**
les tronçons latéraux (501) sont mobiles axialement par rapport à l'armature de façon à modifier le volume intérieur (700),
et **en ce que**
les tronçons latéraux (501) sont reliés au deuxième anneau (505) par au moins un pontet (504, 530).

2. Masque de protection oculaire selon la revendication 1, dans lequel la jupe (500) est réalisée dans un matériau présentant une dureté au moins trois fois inférieure à la dureté de l'armature (100).

3. Masque de protection oculaire selon la revendication 1 ou 2, dans lequel la jupe (500) est réalisée dans un matériau présentant une dureté inférieure à 80 Shore A.

4. Masque de protection oculaire selon l'une quelconque des revendications précédentes, dans lequel le premier anneau (506) présente un contour sensiblement identique au contour de l'armature (100).

5. Masque de protection oculaire selon l'une quelconque des revendications précédentes, dans lequel des dégagements sont ménagés entre les tronçons latéraux du premier anneau et le deuxième anneau.

6. Masque de protection oculaire selon la revendication 5, comprenant du textile respirant obturant les dégagements entre les tronçons latéraux du premier anneau et le deuxième anneau.

7. Masque de protection oculaire selon l'une quelconque des revendications précédentes, dans lequel le au moins un pontet est incliné par rapport à l'axe du masque.

8. Masque de protection oculaire selon l'une quelconque des revendications précédentes, dans lequel au moins un pontet (504) est placé sur le côté supérieur du masque.

9. Masque de protection oculaire selon l'une quelconque des revendications précédentes, dans lequel au moins un pontet (530) est placé sur le côté latéral du masque.

10. Masque de protection oculaire selon l'une quelconque des revendications précédentes, dans lequel les premier et deuxième anneaux (506, 505) sont réalisés d'un seul tenant.

11. Masque de protection oculaire selon l'une quelconque des revendications précédentes, dans lequel le tronçon médian (503) forme un bloc de matière continu entre l'armature (100) et les tronçons latéraux (501).

12. Masque de protection oculaire selon l'une quelconque des revendications précédentes, dans lequel l'écran présente deux zones de vision (380) destinées à venir en vis-à-vis des yeux de l'utilisateur et une partie médiane (390) raccordant lesdites zones de vision, et dans lequel l'armature présente une rainure (101) logeant la bordure de l'écran (300), la rainure présentant une profondeur inférieure à 2,5 mm à la verticale desdites zones de vision de l'écran.

13. Masque de protection oculaire selon l'une quelconque des revendications précédentes, dans lequel le premier anneau présente deux tronçons médians (503) à l'aplomb l'un de l'autre et à partir desquels s'étendent les tronçons latéraux, un tronçon médian étant destiné à venir en vis-à-vis du front de l'utilisateur, l'autre tronçon médian étant destiné à venir en vis-à-vis du nez de l'utilisateur.

## Patentansprüche

1. Augenschutzmaske (1) für die Ausübung von Outdoor-Sportarten, die enthält:
- einen Augenschutzschirm (300), der im Wesentlichen lotrecht zu einer Sichtachse ausgerichtet ist;
- einen Umfangsrahmen (100), an dem der Schirm befestigt ist, wobei der Rahmen eine vom Schirm (300) verschlossene Öffnung (102) begrenzt, wobei der Rahmen aus einem steifen Material hergestellt ist;
- eine Schürze (500), die einen zweiten, am Rahmen (100) befestigten Ring (505) und einen ersten Ring (506) aufweist, von dem ein Mittelabschnitt (503) im Wesentlichen unbeweglich am Rahmen (100) befestigt ist, und der Seitenabschnitte (501) aufweist, die sich vom Mittelabschnitt ausgehend seitlich erstrecken, wobei die Schürze aus biegsamem Material hergestellt ist;
- eine Einfassung (510), die an den Seitenabschnitten (501) befestigt und dazu bestimmt ist, mit dem Gesicht des Benutzers in Kontakt zu kommen;
- wobei das vom Schirm (300), von der Schürze (500) und der Einfassung (510) begrenzte Innenvolumen (700) ein geschlossener Raum ist, sobald die Maske gegen das Gesicht des Benutzers angelegt wird;
**dadurch gekennzeichnet, dass**
die Seitenabschnitte (501) bezüglich des Rahmens axial beweglich sind, um das Innenvolumen (700) zu verändern,
und dass
die Seitenabschnitte (501) durch mindestens einen Steg (504, 530) mit dem zweiten Ring (505) verbunden sind.

2. Augenschutzmaske nach Anspruch 1, wobei die Schürze (500) aus einem Material hergestellt ist, das eine Härte aufweist, die mindestens drei Mal geringer ist als die Härte des Rahmens (100).

3. Augenschutzmaske nach Anspruch 1 oder 2, wobei die Schürze (500) aus einem Material hergestellt ist, das eine Härte von weniger als 80 Shore A aufweist.

4. Augenschutzmaske nach einem der vorhergehenden Ansprüche, wobei der erste Ring (506) einen Umriss aufweist, der im Wesentlichen gleich dem Umriss des Rahmens (100) ist.

5. Augenschutzmaske nach einem der vorhergehenden Ansprüche, wobei Freiräume zwischen den Seitenabschnitten des ersten Rings und dem zweiten Ring ausgespart sind.

6. Augenschutzmaske nach Anspruch 5, die eine atmende Textilie enthält, die die Freiräume zwischen den Seitenabschnitten des ersten Rings und dem zweiten Ring verdeckt.

7. Augenschutzmaske nach einem der vorhergehenden Ansprüche, wobei der mindestens eine Steg bezüglich der Achse der Maske geneigt ist.

8. Augenschutzmaske nach einem der vorhergehenden Ansprüche, wobei mindestens ein Steg (504) an der Oberseite der Maske angeordnet ist.

9. Augenschutzmaske nach einem der vorhergehenden Ansprüche, wobei mindestens ein Steg (530) auf der Seite der Maske angeordnet ist.

10. Augenschutzmaske nach einem der vorhergehenden Ansprüche, wobei der erste und der zweite Ring (506, 505) aus einem Stück hergestellt sind.

11. Augenschutzmaske nach einem der vorhergehenden Ansprüche, wobei der Mittelabschnitt (503) einen durchgehenden Materialblock zwischen dem Rahmen (100) und den Seitenabschnitten (501) bildet.

12. Augenschutzmaske nach einem der vorhergehenden Ansprüche, wobei der Schirm zwei Sichtzonen (380), die dazu bestimmt sind, sich gegenüber den Augen des Benutzers zu befinden, und einen mittleren Bereich (390) aufweist, der die Sichtzonen verbindet, und wobei der Rahmen eine Rille (101) aufweist, die den Rand des Schirms (300) aufnimmt, wobei die Rille eine Tiefe von weniger als 2,5 mm senkrecht zu den Sichtzonen des Schirms aufweist.

13. Augenschutzmaske nach einem der vorhergehenden Ansprüche, wobei der erste Ring zwei Mittelabschnitte (503) aufweist, die lotrecht zueinander sind und von denen ausgehend sich die Seitenabschnitte erstrecken, wobei ein Mittelabschnitt dazu bestimmt ist, sich gegenüber der Stirn des Benutzers zu befinden, während der andere Mittelabschnitt dazu bestimmt ist, sich gegenüber der Nase des Benutzers zu befinden.

## Claims

1. Eye-protection mask (1) for the practice of outdoor sports, comprising:
- an eye-protection lens (300) oriented essentially perpendicular to a visual axis;
- a peripheral frame (100) to which the lens is secured, the frame bounding an opening (102) closed by the lens (300), the frame being made of a rigid material;
- a skirt (500) having a second ring (505) secured to the frame (100) and a first ring (506) of which a median section (503) is secured so as to be essentially immobile on the frame (100), and having lateral sections (501) extending laterally from the median section, the skirt being made of a flexible material;
- padding (510) secured to the lateral sections (501) and designed to be in contact with the face of the user;
- the internal volume (700) bounded by the lens (300), the skirt (500) and the padding (510) being an enclosed space once the mask is placed against the face of the user;
**characterized in that**
the lateral sections (501) are able to move axially with respect to the frame so as to modify the internal volume (700),
and **in that**
the lateral sections (501) are connected to the second ring (505) by at least one bridge (504, 530).

2. Eye-protection mask according to Claim 1, in which the skirt (500) is made of a material having a hardness at least three times less than the hardness of the frame (100).

3. Eye-protection mask according to Claim 1 or 2, in which the skirt (500) is made of a material having a hardness of less than 80 Shore A.

4. Eye-protection mask according to any one of the preceding claims, in which the first ring (506) has a contour that is essentially identical to the contour of the frame (100).

5. Eye-protection mask according to any one of the preceding claims, in which cutouts are created between the lateral sections of the first ring and the second ring.

6. Eye-protection mask according to Claim 5, comprising a breathable textile covering the cutouts between the lateral sections of the first ring and the second ring.

7. Eye-protection mask according to any one of the preceding claims, in which the at least one bridge is inclined with respect to the axis of the mask.

8. Eye-protection mask according to any one of the preceding claims, in which at least one bridge (504) is placed on the upper side of the mask.

9. Eye-protection mask according to any one of the preceding claims, in which at least one bridge (530) is placed on the lateral side of the mask.

10. Eye-protection mask according to any one of the preceding claims, in which the first and second rings (506, 505) are made in one piece.

11. Eye-protection mask according to any one of the preceding claims, in which the median section (503) forms a continuous block of material between the frame (100) and the lateral sections (501).

12. Eye-protection mask according to any one of the preceding claims, in which the lens has two vision zones (380) designed to be in front of the eyes of the user and a median portion (390) connecting said vision zones, and in which the frame has a groove (101) receiving the edge of the lens (300), the groove having a depth of less than 2.5 mm in vertical alignment with said vision zones of the lens.

13. Eye-protection mask according to any one of the preceding claims, in which the first ring has two median sections (503) which are vertically aligned with one another and from which extend the lateral sections, one median section being designed to be in front of the forehead of the user, the other median section being designed to be in front of the nose of the user.
